# EUROPÄISCHE PATENTSCHRIFT

(11) **EP 0 747 486 B1**
(45) Veröffentlichungstag und Bekanntmachung des Hinweises auf die Patenterteilung: **03.11.1999**
(21) Anmeldenummer: 96109088.3
(22) Anmeldetag: 05.06.1996
(51) Int. Cl.: C12P 17/12

(54) **Mikrobiologisches Verfahren zur Herstellung von heteroaromatischen Carbonsäuren mittels Mikroorganismen der Gattung Alcaligenes**
Microbiological method for the synthesis of heteroaromatic caboxylic acids using microorganisms of the genus Alcaligenes
Procédé microbiologique pour la production d'acides carboxyliques hétéroaromatiques en utilisant des microorganismes du genre Alcaligènes

(30) Priorität: 07.06.1995 CH 166495; 13.06.1995 CH 173395
(43) Veröffentlichungstag der Anmeldung: 11.12.1996
(73) Patentinhaber: LONZA AG, CH-3945 Gampel/Wallis (CH)
(72) Erfinder: Kiener, Andreas, Dr., 3930 Visp, Kanton Wallis (CH); Roduit, Jean-Paul, Dr., 3979 Grône, Kanton Wallis (CH); Glöckler, Rainer, 3932 Visperterminen, Kanton Wallis (CH)
(74) Vertreter: Weinhold, Peter, Dr.

(56) Entgegenhaltungen:
- EP-A- 0 504 818

## Beschreibung

Die Erfindung betrifft ein neues mikrobiologisches Verfahren zur Herstellung von heteroaromatischen Carbonsäuren oder deren physiologisch verträglichen Salzen der allgemeinen Formeln 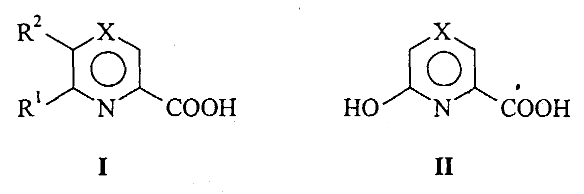 worin R^{1,} R² gleich oder verschieden sind und ein Wasserstoff- oder Halogenatom und X ein Stickstoffatom oder -CH- bedeutet.

Heteroaromatische Carbonsäuren wie beispielsweise 6-Hydroxypikolinsäure, sind wichtige Zwischenprodukte zur Herstellung von Pharmazeutika, wie beispielsweise zur Herstellung von 2-Oxypyrimidin (Berichte der Deutschen Chemischen Gesellschaft 1912, 45, S. 2456 - 2467) oder zur Herstellung von Herbiziden (EP-A 0 447 004).

Generell ist bekannt, dass Mikroorganismen, enthaltend Nitrilhydratasen und Amidasen oder Nitrilasen, Nitrile zu den entsprechenden Säuren umsetzen.
Beispielsweise beschreibt die EP-A 0 187 680 ein mikrobiologisches Verfahren zur Herstellung organischer Säuren wie z. B. Nikotinsäure mittels Mikroorganismen der Gattung *Corynebacterium*, *Nocardia*, *Bacillus, Bacteridium*, *Micrococcus* und *Brevibacterium*. Diese Reaktion wird zwingend in Gegenwart von Lichtenergie durchgeführt. Aus der EP-A 0 444 640 ist ein mikrobiologisches Verfahren zur Herstellung von organischen Säuren, wie z. B. Nikotinsäure, mittels Mikroorganismen der Gattung *Rhodococcus* bekannt. Diese Umsetzung wird zwingend in Gegenwart eines Lactams durchgeführt.

Desweiteren ist bekannt, dass Mikroorganismen der Spezies *Rhodococcus rhodochrous* J1 beispielsweise 2-Cyanpyrazin zur Pyrazincarbonsäure umsetzen (Kobayashi et al., J. of Antibiotics, Vol. 43, No. 10, 1990, S. 1316 - 1320).
Diese Mikroorganismen können jedoch 2-Cyanpyridin nicht zur Picolinsäure umsetzen (Mathew et al., Appl. Environmental Microbiology, Vol. 54, No. 4, 1988, S. 1030 - 1032).

Bekannt ist auch, dass 2-Cyanpyridin-verwertende Mikroorganismen der Gattung *Alcaligenes* 2-Cyanpyridin zu 6-Hydroxypikolinsäure umsetzen (EP-A 0 504 818). Bei diesem Verfahren ist nachteilig, dass die 6-Hydroxypikolinsäure nur in mässiger Ausbeute gebildet wird.

Aufgabe der vorliegenden Erfindung war es, ein wirtschaftlicheres mikrobiologisches Verfahren zur Herstellung von heteroaromatischen Carbonsäuren oder deren physiologisch verträglichen Salzen wie Pyrazincarbonsäure, Pikolinsäure oder Chrompikolinat mittels Mikroorganismen der Gattung Alcaligenes zur Verfügung zu stellen, wobei die gebildeten Carbonsäuren oder deren physiologisch verträgliche Salze in guter Ausbeute gebildet werden.
Diese Aufgabe wurde mit dem Verfahren gemäss Anspruch 1 gelöst.

Erfindungsgemäss wird das Verfahren derart durchgeführt, dass man als Substrat ein heteroaromatisches Nitril der allgemeinen Formeln 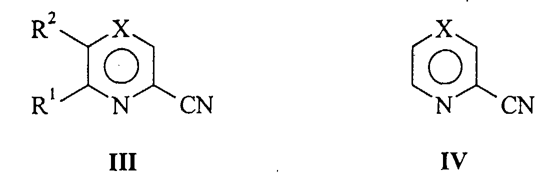 worin X, R¹ und R² die genannte Bedeutung haben mittels 2-Cyanpyridin-verwertenden Mikroorganismen der Gattung *Alcaligenes*, die vor der Biotransformation in Gegenwart einer Dicarbonsäure, Tricarbonsäure oder eines Kohlenhydrats angezüchtet wurden, zu heteroaromatischen Carbonsäuren gemäss Formel I oder II umsetzt. Die heteroaromatischen Carbonsäuren werden dann gegebenenfalls in physiologisch verträgliche Salze überführt. Als physiologisch verträgliche Salze dieser Carbonsäuren werden im folgenden bspw. Chrom-, Calcium- oder Ammoniumsalze verstanden.

Vor der eigentlichen Biotransformation werden die für das Verfahren verwendeten Mikroorganismen der Gattung *Alcaligenes* üblicherweise kultiviert (angezüchtet) und deren wirksame Enzyme zweckmässig mit 2-Cyanpyridin induziert.
Für Anzucht und Induktion kann 2-Cyanpyridin in einer Konzentration von 0,01 bis 20 Gew.%, vorzugsweise in einer Konzentration von 0,1 bis 1 Gew.% verwendet werden.

Unter einer Dicarbonsäure wird im folgenden Fumarsäure, Bernsteinsäure, Äpfelsäure, Glutarsäure, Malonsäure bzw. deren Salze und Derivate wie Ester verstanden.

Unter einer Tricarbonsäure wird im folgenden Citronensäure, Isocitronensäure bzw. deren Salze und Derivate wie Ester verstanden. Als Salze und Derivate dieser Dicarbonsäuren und Tricarbonsäuren können Fumarat, Malat, Malonat, Oxalacetat, Citrat, Aconitat, Isocitrat, 2-Oxoglutarat, Succinat oder Succinyl-CoA verwendet werden. Vorzugsweise wird Fumarat, Malonat oder Succinat verwendet.

Als Kohlenhydrate werden im folgenden Monosaccharide wie Glucose, Disaccharide wie Saccharose, Trehalose oder Maltose, Trisaccharide wie Raffinose, Zuckeralkohole wie Glycerin verstanden. Vorzugsweise wird als Kohlenhydrat Glycerin eingesetzt.

Zweckmässig wird die Dicarbonsäure, Tricarbonsäure bzw. das Kohlenhydrat in einer Konzentration von 0,1 bis 20 Gew.%, vorzugsweise in einer Konzentration von 0,5 bis 5 Gew.% angewendet.

Als Anzuchtsmedium können die in der Fachwelt üblichen Medien verwendet werden, wie beispielsweise das Mineralsalzmedium gemäss Kulla et al. (Arch. Microbiol., 135, 1 - 7, 1983), niedermolare Phosphatpuffer oder das gemäss Tabelle 1. Vorzugsweise wird das in Tabelle 1 beschriebene angewendet.

Nach der Anzuchtsphase bzw. vor der eigentlichen Substratzugabe werden die Mikroorganismen entweder mittels üblichen Trennverfahren geerntet oder das Substrat wird direkt zu den Mikroorganismen gegeben.

Die für die Biotransformation eingesetzten Substrate, die heteroaromatischen Nitrile der Formeln III und IV, wie z. B. 2-Cyanpyridin, sind käufliche Verbindungen.

In den allgemeinen Formeln I bis IV bedeutet X ein Stickstoffatom oder -CH-, vorzugsweise -CH-. Die Reste R¹ und R² sind gleich oder verschieden und bedeuten Wasserstoff oder Halogen wie Fluor, Chlor, Brom oder Jod.
Mögliche Substrate sind demzufolge 2-Cyanpyridin, 6-Chlor-2-cyanpyridin, 5,6-Dichlor-2-cyanpyridin, 2-Cyanpyrazin, 6-Chlor-2-cyanpyrazin, 5-Brom-6-chlor-2-cyanpyrazin. Zweckmässig werden als Substrate 2-Cyanpyridin, 2-Cyanpyrazin oder 6-Chlor-2-cyanpyridin verwendet.

Das Substrat kann für die Biotransformation einmalig oder kontinuierlich zugegeben werden. Zweckmässig erfolgt die Substratzugabe so, dass die Substratkonzentration im Medium 20 Gew.%, vorzugsweise so, dass die Substratkonzentration 10 Gew.% nicht übersteigt.

Die Biotransformation, welche üblicherweise mit ruhenden Zellen durchgeführt wird, wird zweckmässig mit den aus der EP-A 0 504 818 bekannten 2-Cyanpyridin-verwertenden Mikroorganismen der Spezies *Alcaligenes faecalis* mit der Bezeichnung DSM 6335 sowie mit deren funktionell aequivalenten Varianten und Mutanten durchgeführt. Diese Mikroorganismen wurden am 03.01.1991 bei der Deutschen Sammlung von Mikroorganismen und Zellkulturen GmbH, Mascheroder Weg 1b, D-38124 Braunschweig,gemäss Budapester Vertrag hinterlegt.

Unter "funktionell aequivalenten Varianten und Mutanten" werden Mikroorganismen verstanden, die im wesentlichen dieselben Eigenschaften und Funktionen wie die Ursprungsmikroorganismen besitzen. Derartige Varianten und Mutanten können zufällig z. B. durch UV-Bestrahlung gebildet werden.

Zur Biotransformation können die gleichen Medien wie die zur Anzucht der Mikroorganismen verwendet werden.
Die Biotransformation kann auch in An- oder Abwesenheit der zuvor beschriebenen Dicarbonsäuren, Tricarbonsäuren bzw. Kohlenhydrate erfolgen.

Der pH-Wert liegt zweckmässig in einem Bereich von 4 bis 10, vorzugsweise in einem Bereich von 5 bis 9. Die Biotransformation kann bei einer Temperatur von 10 bis 50 °C, vorzugsweise bei einer Temperatur von 20 bis 40 °C durchgeführt werden.

Nach einer üblichen Umsetzungszeit von 6 bis 100 Stunden können dann die entsprechenden Carbonsäuren gemäss Formel I oder II durch übliche Aufarbeitungsmethoden wie z. B. durch Ansäuern erhalten werden. Die Carbonsäuren können auch in Form von Salzen, wie beispielsweise als Ammonium- oder Chrom-Salz, isoliert werden.

Werden als heteroaromatische Carbonsäuren in Position 6 hydroxylierte heteroaromatische Carbonsäuren (allgemeine Formel II) hergestellt, wird die Biotransformation zweckmässig unter aeroben Bedingungen durchgeführt. Wird jedoch eine nicht hydroxylierte heteroaromatische Carbonsäure wie z. B. Pikolinsäure hergestellt, wird die Biotransformation zweckmässig unter anaeroben Bedingungen durchgeführt.

### Beispiele:

### Beispiel 1

### Herstellung von 6-Hydroxypikolinsäure

Für die Herstellung von 6-Hydroxypikolinsäure mit dem Stamm *Alcaligenes faecalis* DSM 6335 wurden folgende Bedingungen gewählt. Verwendung fand ein 7,5 1 Fermenter mit 5 1 Arbeitsvolumen. *Alcaligenes faecalis* DSM 6335 wurde in einem Mineralsalzmedium (Tabelle 1) mit Natriumfumarat als einziger Kohlenstoff- und Energiequelle und 2-Cyanpyridin als Induktor bei 30 °C, 600 rpm und einem pH von 7,0 angezogen. Die Belüftungsrate betrug dabei ca. 3 l/min. Die Zugabe des Natriumfumarats erfolgte dabei pO₂-gesteuert bei einem pO₂ von >30%. Verwendung fand eine 20%ige Stocklösung von Natriumfumarat versetzt mit 0,5% 2-Cyanpyridin. Die Zellen wurden bis zu einer optischen Dichte, gemessen bei 650 nm (OD₆₅₀), von 16 innerhalb von 23 Stunden angezogen, ehe die Biotransformation gestartet wurde. Für die Wachstumsphase wurden ca. 160 g Natriumfumarat in Form einer 20%igen Lösung (ca. 800 ml) verbraucht.

Während der aeroben Biotransformation von 2-Cyanpyridin zu 6-Hydroxypikolinsäure wurde auf die Zugabe einer Kohlenstoff- und Energiequelle verzichtet. Die Biotransformation erfolgte mit ruhenden Zellen.
Die Zugabe des 2-Cyanpyridins erfolgte limitierend mittels einer Pumpe. Die Pumprate wurde dabei "online" mittels HPLC überwacht. Die Konzentration des Zwischenproduktes Pikolinsäure, dessen Bildungsrate ca. 2,5 mal höher ist als die Umsetzungsrate von Pikolinsäure zu 6-Hydroxypicolinsäure (10 g/l·h zu 4 g/l·h), wurde auf Werte <2g /1 limitiert, da sonst die Umwandlung von Pikolinsäure zu 6-Hydroxypicolinsäure inhibiert wurde.
Da 2-Cyanpyridin bei Raumtemperatur ein Feststoff ist, war es notwendig, das Vorlagegefäss enthaltend 2-Cyanpyridin auf 50 °C zu erwärmen, damit 2-Cyanpyridin in flüssiger Form zugegeben werden konnte.
Mit diesem Verfahren war es möglich, innerhalb von 31 Stunden 75 g/l 6-Hydroxypikolinsäure herzustellen.
Das Zwischenprodukt Pikolinsäure konnte am Ende der Biotransformation nicht mehr nachgewiesen werden.

Zur Isolation der 6-Hydroxypikolinsäure wurden die Zellen mittels Filtration abgetrennt. Danach wurde die zellfreie Lösung auf 60 °C aufgeheizt und mit konz. Schwefelsäure auf einen pH von 2 - 2,5 angesäuert. Bei diesem pH fiel die 6-Hydroxypikolinsäure aus der Lösung aus.

Danach wurde langsam auf 4 °C unter Rühren abgekühlt, filtriert, der Rückstand mit entmineralisiertem Wasser gewaschen und getrocknet (100 mbar, 55 °C).
In der Mutterlauge verblieben dabei ca. 2 g/l 6-Hydroxypikolinsäure. Die Ausbeute betrug 87% bezogen auf eingesetztes 2-Cyanpyridin.

**Tabelle 1:**

| **Zusammensetzung:** | **Konzentration (g/l):** |
|---|---|
| Fumarsäure-Dinatriumsalz | 10 |
| Hefeextrakt | 1 |
| MgCl₂∗6H₂O | 0,8 |
| Na₂SO₄ | 0,25 |
| (NH₄)₂SO₄ | 1,0 |
| NH₄Cl | 2,33 |
| NaCl | 0,2 |
| CaCl₂∗2H₂O | 0,16 |
| MnSO₄ | 1,8∗10⁻² |
| H₃BO₃ | 3∗10⁻² |
| NiCl₂ | 2∗10⁻³ |
| NaMoO₄ | 3∗10⁻³ |
| FeSO₄∗7H₂O | 0,3 |
| Na₂EDTA∗2H₂O | 0,75 |
| 2-Cyanpyridin | 1 |
| KH₂PO₄ | 0,4 |
| Na₂HPO₄ | 0,96 |

### Beispiel 2

### Herstellung von Pikolinsäure

Die Anzucht der Biomasse erfolgte wie in Beispiel 1 beschrieben. Die Bildung von Pikolinsäure erfolgte unter strikt anaeroben Bedingungen.
Für die Biotransformation wurde eine 500 ml Glasflasche mit Gummiseptum, gefüllt mit 400 ml Biomasse der OD₆₅₀ = 20, verwendet. Inkubiert wurde bei 30 °C.
Bevor mit der Biotransformation begonnen wurde, wurde der Ansatz mit reinem Stickstoff anaerob gestellt. Hierzu wurde der Ansatz für ca. 30 Minuten über Kanülen unter Rührung mit Stickstoff (50 mbar Überdruck) begast, um den Sauerstoff quantitativ auszutreiben. Um Sauerstoffeintritt während der Biotransformation oder bei der Zugabe von 2-Cyanpyridin auszuschliessen, wurde die Begasung während der Biotransformation aufrecht erhalten (ca. 10 mbar Überdruck).
Die Zugabe von 2-Cyanpyridin erfolgte in 12 Schritten zu je 10 g/l jeweils nach Ablauf einer Stunde. Die Zugabe kann jedoch auch kontinuierlich erfolgen. Mittels HPLC wurde geprüft, ob 2-Cyanpyridin vor Zugabe einer weiteren Portion vollständig zu Pikolinsäure umgesetzt wurde. Während der Biotransformation konnte keine Bildung von Pikolinsäureamid nachgewiesen werden.
Mit diesem Verfahren war es möglich, innert 26 Stunden ca. 150 g/l Pikolinsäure herzustellen. 6-Hydroxypikolinsäure wurde dabei nicht gebildet.

Zur Isolation wurde die zellfreie Pikolinsäurelösung mit CaCl₂ / H₂SO₄ ausgefällt. Dazu wurde die zellfreie Pikolinsäurelösung aus Beispiel 2 3fach verdünnt und unter Rühren mit 0,5 Äquivalenten CaCl₂ pro Äquivalent Pikolinsäure versetzt, nachdem die zellfreie Fermentationslösung auf 90 °C vorgeheizt wurde. Der entstehende Calcium-Pikolinsäure-Komplex fiel dabei sofort aus. Der entstandene Komplex wurde auf 4 °C unter Rühren abgekühlt, über eine Glasfritte (Porosität 3) abfiltriert und mit entmineralisiertem Wasser gewaschen.
Der Filterkuchen wurde in entmineralisiertem Wasser aufgeschlämmt und mit konz. Schwefelsäure bis zu einem pH von 2,5 angesäuert. Dabei wurde die Pikolinsäure aus dem Komplex herausgelöst und gleichzeitig bildete sich unlösliches Calciumsulfat. Da die freie Pikolinsäure sehr gut wasserlöslich ist, konnte Calciumsulfat via Filtration abgetrennt werden. Die Pikolinsäurelösung wurde zur Trockene eingeengt und analysiert. Die Rohausbeute betrug ca. 70% mit einer Reinheit von 86% nach Titration. Der Wassergehalt lag bei 0,7%, gemessen nach der Karl-Fischer-Methode.

### Beispiel 3

### Herstellung von Chrom(III)-pikolinat

Zu einer in einem 500 ml Kolben vorgelegten Ammoniumpikolinat-Lösung (271,4 g; 0,325 mol; 16,8%), pH 7,1 wurde bei 73 °C wässrige Chromtrichlorid-Hexahydrat-Lösung (23,95 g, 0,09 mol Cr in 63 ml Wasser) über einen Zeitraum von 3,5 h zugetropft. Die erhaltene violette Lösung wurde noch 1 h weitergerührt, dann langsam auf 3 °C abgekühlt. Nach dem Absetzten des gebildeten roten Feststoffes wurde die obere blaue Phase abdekantiert. Der Feststoff wurde mit 100 ml Wasser während 30 Min. aufgeschlämmt und anschliessend wieder abdekantiert. Nach einer zweiten Aufschlämmung mit 50 ml Wasser (30 Min.) wurde der Feststoff abgenutscht und bei 50 °C unter Vakuum getrocknet.
Es wurden 33,64 g dunkelrote Kristalle erhalten (90% Ausbeute).

### Beispiel 4

### Anzucht von Alcaligenes faecalis DSM 6335 mit unterschiedlichen Kohlenstoffquellen

Zur Anzucht von *Alcaligenes faecalis* (DSM 6335) wurden 300 ml Erlenmeyerkolben mit 100 ml A+N-Medium (Tabelle 1 ohne Fumarsäure-Dinatriumsalz) verwendet. Das Medium wurde zusätzlich mit 2 gl⁻¹ 2-Cyanpyridin und 10 gl⁻¹ der folgenden Kohlenstoffquellen versetzt:
Fumarsäure-Dinatriumsalz
Glycerin
Malonsäure-Dinatriumsalz
Bernsteinsäure-Dinatriumsalz

Die Inkubation erfolgte auf einer Schüttelmaschine bei 30 °C. Nach 16 h Wachstum wurden die Zellen abzentrifugiert und im frischen A+N-Medium (ohne Kohlenstoffquelle) enthaltend 10 gl⁻¹ 2-Cyanpyridin resuspendiert. Die optische Dichte der Zellsuspension gemessen bei 650 nm (OD₆₅₀) betrug 10. Danach wurden die Zellsuspensionen (Totalvolumen 10 - 20 ml) erneut bei 30 °C inkubiert. Die Bildung von 6-Hydroxypicolinsäure wurde spektrophotometrisch durch Messung der Absorption der zellfreien Lösung bei 308 nm verfolgt. Folgende durchschnittlichen Produktivitäten wurden für die Bildung von 6-Hydroxypicolinsäure bestimmt:

| Kohlenstoffquelle | Produktivität (in gl⁻¹h⁻¹) |
|---|---|
| Fumarsäure-Dinatriumsalz | 2,4 |
| Glycerin | 2,0 |
| Malonsäure-Dinatriumsalz | 4,2 |
| Bernsteinsäure-Dinatriumsalz | 0,14 |

### Beispiel 5

### Herstellung von 6-Hydroxypyrazincarbonsäure

Die Anzucht von *Alcaligenes faecalis* (DSM 6335) erfolgte wie in Beispiel 4 mit Fumarsäure als Kohlenstoffquelle. Die gewaschenen Zellen wurden im A+N-Medium enthaltend 10 gl⁻¹ 2-Cyanpyrazin resuspendiert (OD₆₅₀ = 10) und bei 30 °C inkubiert. Die Bildung von 6-Hydroxypyrazincarbonsäure wurde spektrophotometrisch durch Messung der Absorption der zellfreien Lösung bei 320 nm verfolgt. Die Abnahme der Konzentration von 2-Cyanpyrazin (Substrat) konnte durch Messung der Absorption bei 270 nm bestimmt werden. Nach 7 h war die eingesetzte Menge 2-Cyanpyrazin zu 6-Hydroxypyrazincarbonsäure umgesetzt worden.

### Beispiel 6

### Herstellung von 6-Chlorpicolinsäure und Pyrazincarbonsäure

Die Anzucht von *Alcaligenes faecalis* (DSM 6335) erfolgte wie in Beispiel 4 mit Fumarsäure als Kohlenstoffquelle. Die gewaschenen Zellen wurden im A+N-Medium in Glasgefässen resuspendiert (OD₆₅₀ = 10), die mit Gummistopfen verschlossen werden konnten, und mittels Kanülen mit Stickstoff begast, um gelösten Sauerstoff zu entfernen. Danach wurde zu den Zellsuspensionen 2-Cyanpyrazin resp. 6-Chlor-2-cyanpyridin als Substrat bis zu einer Endkonzentration von 10 gl⁻¹ zugegeben und bei 30 °C inkubiert. Nach 3 h waren die Ausgangssubstanzen quantitativ zu den entsprechenden Säuren umgesetzt [Nachweis mit Dünnschichtchromatographie; Kieselgel 60 mit Fluoreszenzindikator, Laufmittel: Chloroform 30 / Ethanol 55 / NH₄OH (25%) 10 / H₂O 5].

## Patentansprüche

1. Mikrobiologisches Verfahren zur Herstellung von heteroaromatischen Carbonsäuren oder deren physiologisch verträglichen Salzen der allgemeinen Formeln 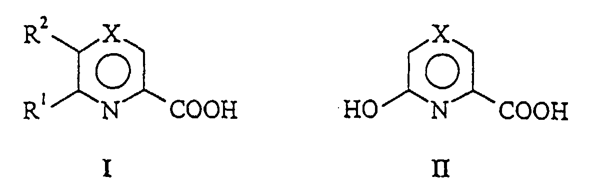 worin R¹ und R² gleich oder verschieden sind und ein Wasserstoff- oder Halogenatom und X ein Stickstoffatom oder -CH- bedeutet, durch Umsetzung von heteroaromatischen Nitrilen der allgemeinen Formeln 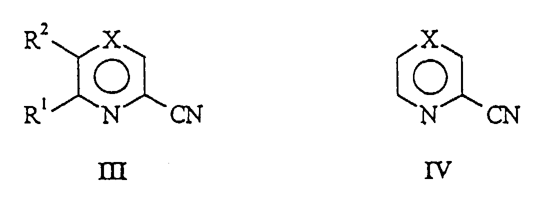 worin R¹, R² und X die genannte Bedeutung haben, mittels 2-Cyanpyridin-verwertenden Mikroorganismen der Gattung *Alcaligenes* zur entsprechenden Carbonsäure und gegebenenfalls anschließende Überführung der erhaltenen Carbonsäure in physiologisch verträgliche Salze, dadurch gekennzeichnet, daß die Mikroorganismen vor der Biotransformation in Gegenwart einer Dicarbonsäure, einer Tricarbonsäure oder eines Kohlehydrats angezüchtet wurden, ausgenommen Verfahren zur Herstellung von 6-Hydroxypikolinsäure durch Umsetzung von 2-Cyanpyridin, worin die Mikroorganismen mit Folsäure als einziger Dicarbonsäure angezüchtet wurden.

2. Verfahren nach Anspruch 1, dadurch gekennzeichnet, daß man die Biotransformation mit Mikroorganismen der Spezies *Alcaligenes faecalis* mit der Bezeichnung DSM 6335 sowie mit deren funktionell aequivalenten Varianten und Mutanten durchführt.

3. Verfahren nach Anspruch 1 oder 2, dadurch gekennzeichnet, daß man die Biotransformation bei einem pH von 4 bis 10 und einer Temperatur von 10 bis 50°C durchführt.

4. Verfahren zur Herstellung von Pikolinsäure oder deren physiologisch verträglichen Salzen durch Umsetzung von 2-Cyanpyridin mittels 2-Cyanpyridin-verwertenden Mikroorganismen der Gattung *Alcaligenes* und gegebenenfalls anschließende Überführung der erhaltenen Pikolinsäure in physiologisch verträgliche Salze, dadurch gekennzeichnet, daß die Umsetzung unter anaeroben Bedingungen erfolgt und die Mikroorganismen vor der Biotransformation in Gegenwart einer Dicarbonsäure angezüchtet wurden.

## Claims

1. Microbiological process for the production of heteroaromatic carboxylic acids or the physiologically compatible salts thereof of the general formulae 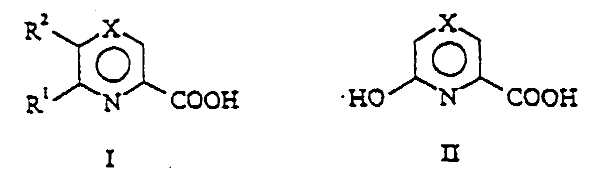 in which R¹ and R² are identical or different and mean a hydrogen or halogen atom and X means a nitrogen atom or -CH-, by transforming heteroaromatic nitriles of the general formulae 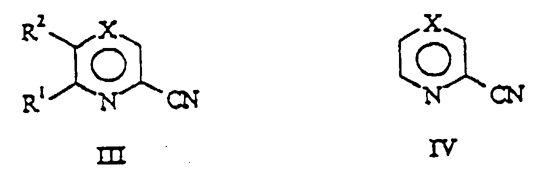 in which R¹, R² and X have the stated meaning, by means of 2-cyanopyridine metabolising microorganisms of the genus *Alcaligenes* to yield the corresponding carboxylic acid and optionally subsequently converting the resultant carboxylic acid into physiologically compatible salts, characterised in that, before the biotransformation, the microorganisms have been cultured in the presence of a dicarboxylic acid, a tricarboxylic acid or a carbohydrate, with the exception of a process for the production of 6-hydroxypicolinic acid by transformation of 2-cyanopyridine, in which the microorganisms have been cultured with folic acid as the sole dicarboxylic acid.

2. Process according to claim 1, characterised in that the biotransformation is performed with microorganisms of the species *Alcaligenes faecalis* with the designation DSM 6335 together with the functionally equivalent variants and mutants thereof.

3. Process according to claim 1 or 2, characterised in that the biotransformation is performed at a pH of 4 to 10 and a temperature of 10 to 50°C.

4. Process for the production of picolinic acid or the physiologically compatible salts thereof by transforming 2-cyanopyridine by means of 2-cyanopyridine metabolising microorganisms of the genus *Alcaligenes* and optionally subsequently converting the resultant picolinic acid into physiologically compatible salts, characterised in that the transformation proceeds under anaerobic conditions and, before the biotransformation, the microorganisms have been cultured in the presence of a dicarboxylic acid.

## Revendications

1. Procédé microbiologique pour la préparation d'acides carboxyliques hétéroaromatiques ou leurs sels physiologiquement compatibles des formules générales 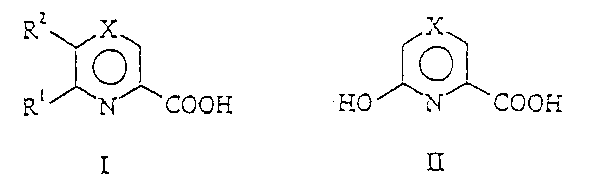 Dans lesquelles R¹ et R² sont identiques ou différents et signifient un atome d'hydrogène ou halogène et X signifie un atome d'azote ou -CH-, par transformation de nitriles hétéroaromatiques des formules générales 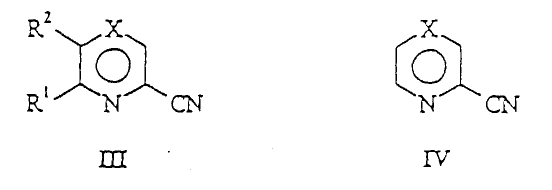 Dans lesquelles R¹, R² et X ont la signification indiquée, au moyen de microorganismes utilisant la 2-cyanopyridine, microorganismes de l'espèce *alcaligenes* en acide carboxylique correspondant et éventuellement transformation consécutive de l'acide carboxylique obtenu en sels physiologiquement compatibles, caractérisé en ce que les microorganismes ont été mis en culture avant la biotransformation en présence d'un acide dicarboxylique, d'un acide tricarboxylique ou d'un hydrate de carbone, à l'exception du procédé pour la préparation de l'acide 6-hydroxypicolinique par mise en réaction de 2-cyanoipyridine, dans lequel les microorganismes ont été mis en culture avec l'acide folique en tant que seul acide dicarboxylique.

2. Procédé selon la revendication 1, caractérisé en ce que l'on effectue la biotransformation avec des microorganismes de l'espèce *Alcaligenes faecalis* portant la désignation DSM 6335 ainsi qu'avec leurs variantes et mutants fonctionnement équivalents.

3. Procédé selon la revendication 1 ou 2, caractérisé en ce que l'on effectue une biotransformation à un pH de 4 à 10 et à une température de 10 à 50°C.

4. Procédé pour la préparation de l'acide picolinique ou de ses sels physiologiquement compatibles par mise en réaction de microorganismes utilisant la 2-cyanopyridine, organismes de l'espèce *Alcaligenes* et éventuellement la transformation consécutive de l'acide picolinique obtenu en sels physiologiquement compatibles, caractérisé en ce que la réaction s'effectue dans des conditions anaérobies et les microorganismes sont mis en culture avant la biotransformation en présence d'un acide dicarboxylique.
